# EUROPÄISCHE PATENTSCHRIFT

(11) **EP 2 294 048 B1**
(45) Veröffentlichungstag und Bekanntmachung des Hinweises auf die Patenterteilung: **17.04.2013**
(21) Anmeldenummer: 09779401.0
(22) Anmeldetag: 05.05.2009
(51) Int. Cl.: C07C 67/03, C07C 69/54

(54) **VERFAHREN ZUR HERSTELLUNG VON (METH)ACRYLSÄUREESTERN**
METHOD FOR THE PRODUCTION OF (METH)ACRYLIC ESTERS
PROCÉDÉ DE PRODUCTION D'ESTERS D'ACIDE (MÉTH)ACRYLIQUE

(30) Priorität: 07.07.2008 DE 102008040221
(43) Veröffentlichungstag der Anmeldung: 16.03.2011
(73) Patentinhaber: Evonik Röhm GmbH, 64293 Darmstadt (DE)
(72) Erfinder: KNEBEL, Joachim, 64665 Alsbach-Hähnlein (DE)
(86) Internationale Anmeldenummer: PCT/EP2009/055367
(87) Internationale Veröffentlichungsnummer: WO 2010/003709

(56) Entgegenhaltungen:
- GB-A- 1 094 998
- GB-A- 1 401 843
- GB-A- 2 037 608

## Beschreibung

Die vorliegende Erfindung betrifft Verfahren zur Herstellung von (Meth)acrylsäureestern.

(Meth)acrylsäureester sind weithin bekannte und vielfach eingesetzte Monomere. Dementsprechend sind vielfältige Methoden bekannt, um diese Verbindungen zu erhalten. Zur Herstellung von Spezial(meth)acrylaten werden vielfach Umesterungsreaktionen durchgeführt, bei denen Methyl(meth)acrylat mit einem entsprechenden Alkohol umgesetzt wird. Zur Verbesserung der Ausbeute und der Selektivität der Reaktion wurden bisher unterschiedliche Katalysatoren eingesetzt.

Beispielsweise beschreibt die Druckschrift DE 28 05 702 die Herstellung von Estern ungesättigter Carbonsäuren. Zur Katalyse der beschriebenen Reaktionen können insbesondere Verbindungen eingesetzt werden, die Zirkonium und/oder Calcium enthalten. Zu den besonders geeigneten Katalysatoren gehört insbesondere Zirkoniumacetylacetonat. Die Umsetzungen führen zu hohen Ausbeuten von ca. 97%, bezogen auf den eingesetzten Alkohol.

Des Weiteren können Säuren oder Basen eingesetzt werden, um die Umesterung zu katalysieren. Derartige Reaktionen werden beispielsweise in CN 1355161, DE 34 23 443 oder EP-A-0 534 666 dargelegt. Zu den basischen Katalysatoren gehört insbesondere Lithiumamid, wie dies beispielhaft in den Druckschriften DE 34 23 443, CA 795814 und US 6,194,530 dargelegt wird.

GB 1 401 843 offenbart die umesterung eine (Meth)acrylesters mit einem Alkohol unter Verwendung eines Ionenaustauschers als Katalysator, dabei wird ein saurer Ionenaustauscher verwendet.

Darüber hinaus wurde vorgeschlagen, die Wirtschaftlichkeit der Herstellung durch eine Zugabe der Ausgangsverbindungen im Verlauf der Reaktion zu verbessern. So beschreibt beispielsweise die Druckschrift US 5,072,027 eine Zugabe von Alkohol und

Methylmethacrylat nach einem hohen Umsatz der zu Beginn der Reaktion eingesetzten Ausgangsverbindungen.

Die zuvor dargelegten Umsetzungen führen bereits zu einem hohen Umsatz und zu reinen Produkten. Aufgrund der hohen wirtschaftlichen Bedeutung von Spezial(meth)acrylaten besteht jedoch das dauerhafte Bestreben die Herstellung dieser Verbindungen weiter zu verbessern.

Nachteilig an den zuvor dargelegten Verfahren ist unter anderem, dass die Katalysatoren aus den Reaktionsmischungen abgetrennt werden müssen. Bei der Abtrennung werden viele der zuvor dargelegten Katalysatoren irreversibel verändert, so dass diese nicht wieder verwendet werden können.

In Anbetracht des Standes der Technik war es nun Aufgabe der vorliegenden Erfindung, ein Verfahren zur Herstellung von (Meth)acrylaten zur Verfügung zu stellen, bei dem das Produkt sehr wirtschaftlich erhalten werden kann. Darüber hinaus sollte der erhaltene (Meth)acrylsäureester nur sehr geringe Mengen an Nebenprodukten und Katalysatorresten enthalten.

Eine weitere Aufgabe der Erfindung bestand darin, ein Verfahren zu schaffen, bei dem (Meth)acrylate sehr selektiv erhalten werden können.

Darüber hinaus war es Aufgabe der vorliegenden Erfindung, Verfahren zur Herstellung von (Meth)acrylaten zur Verfügung zu stellen, die einfach und kostengünstig durchgeführt werden können. Hierbei sollte das Produkt möglichst in hohen Ausbeuten und, insgesamt gesehen, unter geringem Energieverbrauch erhalten werden.

Weiterhin sollte ein Verfahren zur Verfügung gestellt werden, welches ohne aufwendige Abtrennung des Katalysators durchgeführt werden kann.

Gelöst werden diese sowie weitere nicht explizit genannte Aufgaben, die jedoch aus den hierin einleitend diskutierten Zusammenhängen ohne Weiteres ableitbar oder erschließbar sind, durch Verfahren mit allen Merkmalen des Patentanspruchs 1. Zweckmäßige Abwandlungen der erfindungsgemäßen Verfahren werden in den auf Anspruch 1 rückbezogenen abhängigen Ansprüchen unter Schutz gestellt.

Gegenstand der vorliegenden Erfindung ist dementsprechend ein Verfahren zur Herstellung von (Meth)acrylsäureestern, umfassend die Umesterung eines als Ausgangsverbindung eingesetzten Esters mit einem niedrigeren Siedepunkt als der durch Umesterung erhaltenen Ester der (Meth)acrylsäure mit einem Eduktalkohol in Gegenwart eines Katalysators, dadurch gekennzeichnet, dass die Umesterung durch einen basischen Ionenaustauscher katalysiert wird.

Hierdurch gelingt es auf nicht vorhersehbare Weise ein Verfahren zur Herstellung von (Meth)acrylaten zur Verfügung zu stellen, bei dem das Produkt sehr wirtschaftlich erhalten wird. Überraschend enthält das erhaltene Produkt nur sehr geringe Mengen an Nebenprodukten und Katalysatorresten. Der erfindungsgemäß zu verwendende Katalysator kann besonders einfach und vollständig abgetrennt werden. Hierbei kann der Katalysator ohne größeren Aufwand wieder verwendet werden.

Des Weiteren ermöglicht das erfindungsgemäße Verfahren eine besonders selektive Herstellung von (Meth)acrylaten.

Weiterhin kann das erfindungsgemäße Verfahren einfach und kostengünstig durchgeführt werden, wobei das Produkt in hohen Ausbeuten und, insgesamt gesehen, unter geringem Energieverbrauch erhalten werden kann. Darüber hinaus kann das erfindungsgemäße Verfahren auf besonders einfache Weise kontinuierlich durchgeführt werden. Hierdurch kann eine hohe Produktqualität bei sehr geringen Kosten erhalten werden.

Erfindungsgemäß werden (Meth)acrylate hergestellt, wobei der Ausdruck (Meth)acrylat für Methacrylsäureester, Acrylsäureester und Mischungen von beiden steht. (Meth)acrylate sind an sich weithin bekannt. Zu diesen Verbindungen gehören unter anderem (Meth)acrylate, die sich von gesättigten Alkoholen ableiten, wie Hexyl(meth)acrylat, 2-Ethylhexyl(meth)acrylat, Heptyl(meth)acrylat, 2-(tert.-Butylamino)ethyl(meth)acrylat, Octyl(meth)acrylat, 3-iso-Propylheptyl(meth)acrylat, Nonyl(meth)acrylat, Decyl(meth)acrylat, Undecyl(meth)acrylat, 5-Methylundecyl(meth)acrylat, Dodecyl(meth)acrylat, 2-Methyldodecyl(meth)acrylat, Tridecyl(meth)acrylat, 5-Methyltridecyl(meth)acrylat, Tetradecyl(meth)acrylat, Pentadecyl(meth)acrylat, Hexadecyl(meth)acrylat, 2-Methylhexadecyl(meth)acrylat, Heptadecyl(meth)acrylat, 5-iso-Propylheptadecyl(meth)acrylat, 4-tert.-Butyloctadecyl(meth)acrylat, 5-Ethyloctadecyl(meth)acrylat, 3-iso-Propyloctadecyl(meth)acrylat, Octadecyl(meth)acrylat, Nonadecyl(meth)acrylat, Eicosyl(meth)acrylat, Cetyleicosyl(meth)acrylat, Stearyleicosyl(meth)acrylat, Docosyl(meth)acrylat und/oder Eicosyltetratriacontyl(meth)acrylat; (Meth)acrylate, die sich von ungesättigten Alkoholen ableiten, wie z. B. 2-Propinyl(meth)acrylat, Allyl(meth)acrylat, Vinyl(meth)acrylat, Oleyl(meth)acrylat; Cycloalkyl(meth)acrylate, wie Cyclopentyl(meth)acrylat, 3-Vinylcyclohexyl(meth)acrylat, Cyclohexyl(meth)acrylat, Bomyl(meth)acrylat; (Meth)acrylate, die zwei oder mehr (Meth)acryl-Gruppen aufweisen, Glycoldi(meth)acrylate, wie Ethylenglycoldi(meth)acrylat, Diethylenglycoldi(meth)acrylat, Triethylenglycoldi(meth)acrylat, Tetra- und Polyethylenglycoldi(meth)acrylat, 1,3- Butandiol(meth)acrylat, 1,4-Butandiol(meth)acrylat, 1,6-Hexandioldi(meth)acrylat, Glycerindi(meth)acrylat und Dimethacrylate von ethoxyliertem Bisphenol A; (Meth)acrylate mit drei oder mehr Doppelbindungen, wie z.B. Glycerintri(meth)acrylat, Trimethylolpropantri(meth)acrylat, Pentaerythrittetra(meth)acrylat und Dipentaerythritpenta(meth)acrylat.

Zu den besonders bevorzugten (Meth)acrylaten gehören insbesondere 2-Ethylhexylmethacrylat, Ethylenglycoldimethacrylat (2-Methylpropensäure-1,2-ethandiyldiester; CAS-Nummer 97-90-5), ethoxyliertes Bisphenol A-dimethacrylat, 1,3-Butandioldimethacrylat (CAS-Nummer 1189-08-8), 1,4- Butandioldimethacrylat (CAS-Nummer 2082-81-7) und/oder Trimethylolpropantrimethacrylat.

Zur Herstellung der (Meth)acrylate wird erfindungsgemäß ein Alkohol eingesetzt, der hierin auch als Eduktalkohol bezeichnet wird. Die Art des Alkohols ergibt sich hierbei von der beabsichtigten Zielverbindung. Dementsprechend können insbesondere Alkohole mit 5 oder mehr Kohlenstoffatomen, ungesättigte Alkohole und/oder mehrwertige Alkohole eingesetzt werden. Zu den bevorzugten Alkoholen mit 5 oder mehr Kohlenstoffatomen gehören beispielsweise Pentanol, Hexanol, 2-Ethylhexanol, Heptanol, 2-Ethylhexylalkohol, 2-tert.-Butylheptanol, Octanol, 3-iso-Propylheptanol, Nonanol, Decanol, Undecanol, 5-Methylundeanol, Dodecanol, 2-Methyldodecanol, Tridecanol, 5-Methyltridecanol, Tetradecanol, Pentadecanol, Hexadecanol, 2-Methylhexadecanol, Heptadecanol, 5-iso-Propylheptadecanol, 4-tert.-Butyloctadecanol, 5-Ethyloctadecanol, 3-iso-Propyloctadecanol, Octadecanol, Nonadecanol, Eicosanol, Cetyleicosanol, Stearyleicosanol, Docosanol und/oder Eicosyltetratriacontanol. Zu den bevorzugten ungesättigten Alkoholen gehören insbesondere 2-Propin-1-ol, Allylalkohol und Vinylalkohol und/oder Oleylalkohol.

Gemäß einem besonderen Aspekt der vorliegenden Erfindung können einwertige Alkohole eingesetzt werden, die genau eine Hydroxygruppe umfassen.

Besonders bevorzugt werden mehrwertige Alkohole eingesetzt. Mehrwertige Alkohole sind organische Verbindungen mit zwei, drei, vier oder mehr Hydroxygruppen. Zu diesen Verbindungen gehören insbesondere Ethylenglycol, Trimethylolpropan, 1,2-Propandiol, 1,3-Propandiol, 1,3-Butandiol, 1,4-Butandiol, ethoxyliertes Bisphenol A, 1,6-Hexandiol, Pentaerythrit, Polyethylenglycol, insbesondere Polyethylenglycol 400, und/oder Glycerin, wobei Trimethylolpropan besonders bevorzugt ist. Die Verbindungen sind vielfach kommerziell zum Beispiel von BASF AG, der Celanese AG oder der Clariant GmbH erhältlich.

Gemäß der vorliegenden Erfindung wird ein Alkohol mit einem niedrig siedenden Ester der (Meth)acrylsäure umgesetzt. Der Begriff "niedrig siedender Ester" bedeutet, dass der als Ausgangsverbindung eingesetzte Ester einen niedrigeren Siedepunkt aufweist als der durch die Umesterung erhaltene Ester. Bei einem Druck von 10 mbar beträgt die Differenz des Siedepunkts vorzugsweise mindestens 2°C, besonders bevorzugt mindestens 10°C und ganz besonders bevorzugt mindestens 20°C. Besonders geeignete (Meth)acrylate werden insbesondere von Alkoholen mit 1 bis 4 Kohlenstoffatomen gebildet. Zu diesen gehören insbesondere Methanol, Ethanol, n-Propanol, iso-Propanol, n-Butanol und tert.-Bütanol. Besonders bevorzugt wird insbesondere Ethyl(meth)acrylat oder Methyl(meth)acrylat eingesetzt, wobei Methylmethacrylat ganz besonders bevorzugt ist.

Das Gewichtsverhältnis von Eduktalkohol zum niedrig siedenden Ester der (Meth)acrylsäure liegt vorzugsweise im Bereich von 2:1 bis 1:20, besonders bevorzugt 1:2 bis 1:15 und ganz besonders bevorzugt im Bereich von 1:3 bis 1:10.

Die erfindungsgemäße Umesterung wird durch einen basischen Ionenaustauscher katalysiert. Die hierfür basischen Ionenaustauscher sind an sich weithin bekannt, wobei diese ausführlich beispielsweise in Ullmanns Encyclopedia of Industrial Chemistry (6. Auflage) dargelegt sind.

Basische Ionenaustauscher weisen vorzugsweise ein polymeres Gerüst auf, welches durch basische Gruppen modifiziert wurde. Zu den bevorzugten polymeren Gerüsten zählen insbesondere Polymere auf Basis von Styrol und Poly(meth)acrylate, wobei diese Polymere besonders bevorzugt vernetzt sind. Diese Vernetzung kann beispielsweise mit Divinylstyrol erfolgen. Weiterhin können Phenol-Formaldehydharze oder Polyalkylaminharze eingesetzt werden. Zu den bevorzugten basischen Gruppen gehören insbesondere Stickstoff umfassende Gruppen, die aliphatisch oder aromatisch sein können.

So können basische Ionenaustauscher in der aktiven Form beispielsweise Ammoniumgruppen und/oder Pyridiniumgruppen umfassen. Bevorzugte Aminogruppen entsprechen im Allgemeinen der Formel P-V-NR₃⁺, worin P das polymere Gerüst, V eine Bindung oder eine Verbindungsgruppe und R unabhängig für Wasserstoff oder eine Alkylgruppe mit 1 bis 4 Kohlenstoffatomen darstellt. Zu den bevorzugten Alkylgruppen zählen insbesondere die Methylgruppe sowie die Ethylgruppe. Die Verbindungsgruppe kann eine Gruppe mit 1 bis 100, vorzugsweise 1 bis 4 Kohlenstoffatomen darstellen. Vorzugsweise stellt V eine Bindung, eine Methylen-, Ethylen- Propylen- oder Butylengruppe dar.

Die Umesterungsreaktion kann vorzugsweise mit einem stark basischen Ionenaustauscher durchgeführt werden. Stark basische Ionenaustauscher umfassen eine quartäre Stickstoffgruppe, d.h. das Stickstoffatom weist keine Bindung zu einem Wasserstoffatom auf. Dementsprechend umfassen stark basische Ionenaustauscher beispielsweise am Stickstoff alkylierte Pyridiniumgruppen oder quartäre Ammoniumgruppen, wobei beispielsweise in Formel P-V-NR₃⁺ alle Reste R eine Gruppe mit vorzugsweise 1 bis 4 Kohlenstoffatomen bezeichnen.

Ionenaustauscher können hierbei auch verschiedene stickstoffhaltige Gruppen umfassen. Dementsprechend können Ionenaustauscher eingesetzt werden, die primäre, sekundäre, tertiäre und/oder quartäre Aminogruppen aufweisen. Diese Verbindungen zeigen sowohl Eigenschaften stark basischer als auch schwach basischer Ionenaustauscher

Zur Umsetzung wird der Ionenaustauscher im Allgemeinen in eine Form überführt, die bei einer Reaktion mit Wasser zu einer Anhebung des pH-Wertes führt, d.h. der Ionenaustauscher wird mit einem basischen Anion beladen, so dass ein basischer Ionenaustauscher erhalten wird. Zu den bevorzugten basischen Anionen zählen insbesondere Hydroxidionen und Carbonationen.

Bevorzugte Ionenaustauscher weisen in getrockneter Form eine Partikelgröße im Bereich von 0,3 bis 0,8 mm besonders bevorzugt im Bereich von 0,5 bis 0,7 mm auf, die durch Siebanalyse gemessen wird.

Die Austauschkapazität der Ionenaustauscher ist an sich nicht kritisch, wobei diese vorzugsweise im Bereich von 0,5 eq/l bis 1,8 eq/l, besonders bevorzugt im Bereich von 0,7 eq/l bis 1,5 eq/l liegt (trockener Zustand).

Aus Kostengründen und aufgrund ihrer Leistungsfähigkeit sind insbesondere basische Ionenaustauscher auf Basis von Polystyrol bevorzugt, die quartäre Aminogruppen aufweisen.

Die zuvor beschriebenen Anionenaustauscher sind kommerziell unter anderem von Bayer AG unter der Handelsbezeichnung ®Lewatit; von Rohm & Haas Comp./USA unter der Handelsbezeichnung ®Amberlite, ®Ambersep sowie ®Amberlyst und von The Dow Chemical Com./USA unter der Handelsbezeichnung ®Dowex erhältlich. Besonders bevorzugt können insbesondere ®Ambersep 900 und ®Amberlyst A26 zur Umesterung eingesetzt werden.

Die Menge an eingesetztem Katalysator kann in einem weiten Bereich liegen. Von besonderem Interesse sind jedoch Verfahren, bei denen der Anteil an Katalysator, bezogen auf das Gewicht der eingesetzten Reaktionspartner, im Bereich von 0,1 bis 30 Gew.-%, vorzugsweise im Bereich von 1 bis 20 Gew.-% und besonders bevorzugt im Bereich von 5 bis 15 Gew.-% liegt. Dieses Gewicht bezieht sich auf das Trockengewicht des eingesetzten Ionenaustauschers.

Die Umsetzung kann bei Über- oder Unterdruck erfolgen. Gemäß einer besonders zweckmäßigen Abwandlung der vorliegenden Erfindung kann die Umesterung bei einem Druck im Bereich von 200 bis 2000 mbar, besonders bevorzugt im Bereich von 500 bis 1300 mbar durchgeführt werden.

Die Reaktionstemperatur kann, insbesondere in Abhängigkeit des Druckes, ebenfalls in einem weiten Bereich liegen. Gemäß einer bevorzugten Ausführungsform der vorliegenden Erfindung erfolgt die Umsetzung vorzugsweise bei einer Temperatur im Bereich von 50°C bis 140°C, besonders bevorzugt im Bereich von 70°C bis 120°C und ganz besonders bevorzugt 80 bis 110°C.

Überraschend können besondere Vorteile erzielt werden, falls die Temperatur, bei der die Umsetzung erfolgt, im Verlauf der Umsetzung erhöht wird. Gemäß dieser bevorzugten Abwandlung des erfindungsgemäßen Verfahrens kann die Temperatur zu Beginn der Umsetzung, insbesondere bis zu einem Umsatz von 80%, bevorzugt bis zu einem Umsatz von 70%, bezogen auf das Gewicht des eingesetzten Alkohols, vorzugsweise im Bereich von 50°C bis 90°C und gegen Ende der Umsetzung, insbesondere nach einem Umsatz von 80%, bevorzugt nach einem Umsatz von 90%, bezogen auf das Gewicht des eingesetzten Alkohols, im Bereich von 95 °C bis 130°C liegen.

Überraschend ist ferner, dass bei den vorstehend genannten Umsetzungstemperaturen keine merkliche Desaktivierung der Ionentauscher eintritt. Bei den insbesondere in der OH-Form eingesetzten bevorzugt stark basischen Ionenaustauschern wird von den Herstellern eine maximale Gebrauchstemperatur angegeben, die weit unter der Reaktionstemperatur bei der Umesterung liegt. So liegt die maximale Gebrauchstemperatur nach Datenblatt für ®Amberlyst A 26 OH und ®Ambersep 900 OH bei 60°C. Oberhalb dieser Temperaturen soll es zum Abbau der quartären Ammoniumsalzstrukturen durch Aminabspaltung kommen.

Aufgrund der überraschend geringen Desaktivierung des Katalysators kann ein bereits in einer Umesterungsreaktion eingesetzter basischer Ionentauscher als Katalysator in einer weiteren Umsetzung eingesetzt werden. Vorzugsweise kann ein gebrauchter basischer Ionentauscher durch Behandeln mit einer wässrigen Alkalimetallhydroxid- oder -carbonatlösung regeneriert werden.

Das erfindungsgemäße Verfahren kann in Substanz, d.h. ohne Verwendung eines weiteren Lösungsmittels durchgeführt werden. Falls gewünscht kann auch ein inertes Lösungsmittel eingesetzt werden. Hierzu gehören unter anderem Benzol, Toluol, n-Hexan, Cyclohexan und Methylisobutylketon (MIBK) und Methylethylketon (MEK).

Vorzugsweise findet die Umesterung unter Ausschluss von Wasser statt, wobei geringe Mengen an Wasser vielfach toleriert werden können. Gemäß diesem Aspekt der vorliegenden Erfindung umfasst die Reaktionsmischung vorzugsweise höchstens 0,5 Gew.-% Wasser, besonders bevorzugt höchstens 0,05 Gew.-% Wasser.

Bei einer besonders zweckmäßigen Varianten der erfindungsgemäßen Umesterung werden sämtliche Komponenten, wie beispielsweise der Alkohol, der Methacrylsäureester sowie der Katalysator, gemischt, wonach diese Reaktionsmischung bis zum Sieden erwärmt wird. Anschließend kann der frei werdende Alkohol, beispielsweise Methanol oder Ethanol, destillativ, gegebenenfalls azeotrop mit Methylmethacrylat oder Ethylmethacrylat, aus der Reaktionsmischung entfernt werden.

Die Reaktionszeiten sind unter anderem von den gewählten Parametern, wie zum Beispiel Druck und Temperatur, abhängig. Sie liegen aber im Allgemeinen im Bereich von 1 bis 24 Stunden, bevorzugt von 3 bis 20 Stunden und ganz besonders bevorzugt 4 bis 12 Stunden. Bei kontinuierlichen Verfahren liegen die Verweilzeiten im Allgemeinen im Bereich von 0,5 bis 24 Stunden, bevorzugt von 1 bis 12 Stunden und ganz besonders bevorzugt 2 bis 4 Stunden. Weitere Hinweise in Bezug auf die Reaktionszeiten kann der Fachmann den beigefügten Beispielen entnehmen.

Vorzugsweise kann die Reaktion unter Rühren stattfinden, wobei die Rührgeschwindigkeit besonders bevorzugt im Bereich von 50 bis 2000 Upm, ganz besonders bevorzugt im Bereich von 100 bis 500 Upm liegen kann.

Der pH-Wert kann in einem weiten Bereich liegen. Zweckmäßig kann die Umsetzung bei einem pH-Wert im Bereich von 8 bis 14, vorzugsweise 9 bis 13 durchgeführt werden. Zur Bestimmung des pH-Wertes kann ein Teil der Reaktionsmischung in einen Überschuss an Wasser (beispielsweise die 10fache Gewichtsmenge) gegeben werden. Anschließend wird der pH-Wert der wässrigen Phase bei 25°C auf konventionelle Weise bestimmt.

Um eine unerwünschte Polymerisation der Methacrylate zu verhindern, können bei der Umsetzung Polymerisationsinhibitoren eingesetzt werden. Diese Verbindungen, wie beispielsweise Hydrochinone, Hydrochinonether, wie Hydrochinonmonomethylether oder Di-tert-butylbrenzcatechin, Phenothiazin, N,N'-(Diphenyl)-p-phenylendiamin, 4-Hydroxy-2,2,6,6-tetramethylpiperidin-1-oxyl, p-Phenylendiamin, Methylenblau oder sterisch gehinderte Phenole, sind in der Fachwelt weithin bekannt. Diese Verbindungen können einzeln oder in Form von Mischungen eingesetzt werden und sind im Allgemeinen kommerziell erhältlich. Die Wirkung der Stabilisatoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Vorzugsweise werden insbesondere Phenole als Polymerisationsinhibitor eingesetzt. Besonders überraschende Vorteile können bei Verwendung von Hydrochinonmonomethylether erzielt werden. Bezogen auf das Gewicht der gesamten Reaktionsmischung kann der Anteil der Inhibitoren einzeln oder als Mischung im Allgemeinen 0,01 - 0,5 % (wt/wt) betragen.

Diese Polymerisationsinhibitoren können vor oder zu Beginn der Reaktion in die Reaktionsmischung gegeben werden. Darüber hinaus können auch Teile der beigefügten Polymerisationsinhibitoren während der Umesterung hinzu gegeben werden. Von besonderem Interesse sind hierbei insbesondere Verfahren bei denen ein Teil des Polymerisationsinhibitors über den Kolonnenrücklauf zugefügt wird. Besonders zweckmäßig sind unter anderem Mischungen, die Methylmethacrylat, Hydrochinonmonomethylether und 4-Hydroxy-2,2,6,6-tetramethylpiperidin-1-oxyl enthalten. Durch diese Maßnahme kann insbesondere eine unerwünschte Polymerisation innerhalb der Destillationskolonne vermieden werden.

Zur Inhibierung kann des Weiteren Sauerstoff verwendet werden. Hierbei kann dieser zum Beispiel in Form von Luft eingesetzt werden, wobei die Mengen vorteilhaft so dosiert werden, dass der Gehalt in der Gasphase über dem Reaktionsgemisch unterhalb der Explosionsgrenze bleibt. Besonders bevorzugt sind hierbei Luftmengen im Bereich von 0,05 bis 0,5 I pro Stunde und Mol Alkohol. Bei Chargenverfahren kann sich diese Menge auf die ursprünglich eingesetzte Menge an Alkohol beziehen. Bei kontinuierlichen Verfahren kann sich diese Menge auf die zugeführte Menge an Alkohol beziehen. Ebenso können Inertgas-Sauerstoff-Gemische, z.B. Stickstoff-Sauerstoff- oder Argon-Sauerstoff-Gemische eingesetzt werden.

Gemäß einer besonderen Ausgestaltung der vorliegenden Erfindung kann zur Inhibierung eine Kombination von Sauerstoff mit mindestens einem Phenol, vorzugsweise Hydrochinonmonomethylether, eingesetzt werden.

Der aus dem eingesetzten (Meth)acrylat freigesetzte Alkohol, beispielsweise Methanol und/oder Ethanol, kann bevorzugt durch Destillation abgetrennt werden. Hierbei kann vorteilhaft beispielsweise eine Mischung abgetrennt werden, die Methylmethacrylat und Methanol enthält. Überraschend kann mit Vorteil ein Teil der abgetrennten Mischung in den folgenden Ansatz zurückgeführt werden. Gemäß dieser Abwandlung kann der zurückführbare Anteil der abgetrennten Mischung zu Ende der Reaktion, insbesondere nach einem Umsatz von 80%, bevorzugt nach einem Umsatz von 90% des eingesetzten Alkohols, erhalten werden. Beispielsweise kann der Anteil der zurückgeführten Mischung zu Beginn des folgenden Ansatzes im Bereich von 10 bis 50%, bezogen auf die Gesamteinwaage an umzuesterndem Methacrylsäureester liegen.

Die Umesterung kann sowohl kontinuierlich als auch chargenweise durchgeführt werden. Eine kontinuierliche Umsetzung kann bevorzugt in Anlagen mit mehreren Reaktoren ausführt werden, wobei u.a. die Reaktionstemperatur verändert und Alkohol aus dem Reaktionssystem abgetrennt werden kann, der aus dem niedrig siedenden (Meth)acrylsäureester freigesetzt wird.

Von besonderem Interesse sind insbesondere Semibatch-Verfahren, bei denen ein Teil der Reaktionsmischung vorgelegt wird. In weiteren Schritten oder kontinuierlich kann nach Beginn der Umsetzung niedrig siedender Ester der (Meth)acrylsäure in die Reaktionsmischung hinzugefügt werden.

Gemäß einer besonderen Abwandlung der vorliegenden Erfindung kann das in der Reaktionsmischung vorhandene Molverhältnis von niedrig siedendem Ester der (Meth)acrylsäure zu Eduktalkohol während der Umsetzung durch Zugabe von niedrig siedendem Ester der (Meth)acrylsäure erhöht werden. Der Ausdruck "Zugabe von niedrig siedendem Ester" bedeutet, dass von einer externen Quelle diese Verbindung zugeführt wird. Ein reiner Rückfluss, der innerhalb der Destillationskolonne erfolgt, stellt dementsprechend keine Zugabe dar. Von besonderem Interesse sind insbesondere Verfahren in denen das in der Reaktionsmischung vorhandene Molverhältnis des Anteils an niedrig siedendem Ester der (Meth)acrylsäure zum Anteil des zur Umesterung eingesetzten Alkohols während der Umsetzung durch Zugabe von niedrig siedenden Ester der (Meth)acrylsäure um mindestens 10%, besonders bevorzugt um mindestens 40% und ganz besonders bevorzugt um mindestens 100% erhöht wird. Beispielsweise kann das Molverhältnis des während der Umsetzung zugegebenen niedrig siedenden Esters zur Menge des zu Beginn der Reaktion eingesetzten niedrig siedenden Esters im Bereich von 1:5 bis 2:1 liegen, besonders bevorzugt 1:3 bis 1:1,5 liegen. Zweckmäßig kann das Gewichtsverhältnis von Eduktalkohol zum niedrig siedenden Ester der (Meth)acrylsäure zu Beginn der Reaktion vorzugsweise im Bereich von 1:2 bis 1:8, besonders bevorzugt 1:2,5 bis 1:6 und ganz besonders bevorzugt im Bereich von 1:3 bis 1:4 liegen. Durch Zugabe von niedrig siedendem Ester während der Umsetzung kann dieses Verhältnis beispielsweise auf 1:3 bis 1:20, bevorzugt 1:4 bis 1:15 und ganz besonders bevorzugt 1:6 bis 1:10 erhöht werden. Diese Werte beziehen sich jeweils auf den in der Reaktionsmischung vorhandenen Eduktalkohol. Der bereits zum Produktester umgesetzte Anteil des Eduktalkohols kann vielfach durch den Anteil an abdestilliertem Alkohol bestimmt werden, der durch die Reaktion freigesetzt wurde. Darüber hinaus kann der in der Reaktionsmischung befindliche Anteil an Eduktalkohol durch Gaschromatographie bestimmt werden.

Gemäß einer besonderen Ausgestaltung kann die Menge an zugegebenen niedrig siedenden Ester der (Meth)acrylsäure durch die aus der Reaktionsmischung abgetrennte Menge an freigesetztem Alkohol gesteuert werden. Zur Steuerung kann beispielsweise die Temperatur eingesetzt werden, die sich in der Kolonne bei einer geeigneten Höhe einstellt. Auch kann das Rücklaufverhältnis über die Temperatur in der Kolonne während der destillativen Entfernung des aus dem Edukt freigesetzen Alkohols eingestellt werden. Wird beispielsweise ein Gemisch aus der Reaktionsmischung abgetrennt, das Methanol und Methylmethacrylat umfasst, so kann über einen längeren Zeitraum eine Temperatur von ca. 75 bis 85°C vorgegeben werden, oberhalb von der kein Destillat abgezogen wird. Erst unterhalb dieser Temperatur wird eine entsprechende Menge eines Gemisches abgetrennt. Hierdurch kann erreicht werden, dass ein relativ hohes Verhältnis von niedrig siedendem (Meth)acrylat zu Eduktalkohol über einen langen Zeitraum aufrecht erhalten bleibt, ohne dass der Reaktionsmischung allzu große Mengen an niedrig siedendem (Meth)acrylat zugeführt werden müssen.

Überraschend kann durch eine Zugabe von niedrig siedendem Ester während der Umesterung eine besonders hohe Ausnutzung des Kesselvolumens erzielt werden, so dass große Mengen an Spezial(meth)acrylaten auch mit relativ kleinen Anlagen erzeugt werden können. Weiterhin kann die Menge an Spezial(meth)acrylat, die pro Ansatz erzeugt wird, erhöht werden, so dass weitere Vorteile erzielt werden können, da die Kosten zur Durchführung der Reaktion, bezogen auf die erhaltene Menge an Produkt, sinken.

Zweckmäßig kann die Zugabe des niedrig siedenden Ester der (Meth)acrylsäure über einen Zeitraum erfolgen, der mindestens 30%, bevorzugt mindestens 50 % und ganz besonders mindestens 70% der Reaktionszeit entspricht. Hierbei kann die Zugabe innerhalb dieses Zeitraums in Schritten erfolgen, wobei die erste Zugabe den Beginn dieses Zeitraums und die letzte Zugabe den Endpunkt dieses Zeitraums festlegt. Vorzugsweise erfolgt die Zugabe in mindestens drei bevorzugt mindestens 5 und ganz besonders bevorzugt mindestens 10 Schritten. Darüber hinaus kann diese Zugabe auch kontinuierlich erfolgen.

Von besonderem Interesse sind unter anderem Chargenverfahren, bei denen während der Umesterung Methyl(meth)acrylat zugegeben wird. Diese Ausführungsform ist beispielsweise von Vorteil, falls Methyl(meth)acrylat zusammen mit Methanol aus der Reaktionsmischung entfernt wird. Vorzugsweise kann das Gewichtsverhältnis der Menge an während der Umesterung zugegebenem Methyl(meth)acrylat zur Menge an abgetrenntem Methanol-Methyl(meth)acrylat-Gemisch im Bereich von 2:1 bis 1:2, besonders bevorzugt 1,5:1 bis 1:1,5 liegen.

Bei Chargenverfahren kann gegen Ende der Reaktion überschüssiges Edukt, insbesondere der nicht umgesetzte Ester der (Meth)acrylsäure durch Destillation abgetrennt werden. Auch dieser kann im nächsten Batch ohne weitere Reinigung wieder eingesetzt werden.

Das zu Beginn der Umsetzung erhaltene Destillat, welches beispielsweise große Mengen an Methanol oder Ethanol umfassen kann, kann ebenfalls recycliert werden, beispielsweise durch Einarbeitung in eine im Verbund betriebene Anlage zur Herstellung des umzuesternden (Meth)acrylsäureesters.

Eine geeignete Anlage zur Durchführung der vorliegenden Umesterung kann beispielsweise einen Rührkesselreaktor mit Rührwerk, Dampfheizung, Destillationskolonne und Kondensator umfassen. Derartige Anlagen sind an sich bekannt und beispielsweise in Ullmanns Encyclopedia of Industrial Chemistry (6. Auflage), Verlag Wiley-VCH, Weinheim 2003, Band 10, Seite 647, beschrieben. Die Größe der Anlage ist von der herzustellenden Menge an (Meth)acrylat abhängig, wobei das vorliegende Verfahren sowohl im Labormaßstab als auch in großtechnischem Maßstab durchgeführt werden kann. Gemäß einem besonderen Aspekt kann der Rührkesselreaktor dementsprechend ein Kesselvolumen im Bereich von 1m³ bis 30 m³, bevorzugt 3 m³ bis 20 m³ aufweisen. Das Rührwerk des Reaktorkessels kann insbesondere in Form eines Ankerrührers, Impellers, Schaufel- oder Inter-MIG-rührers ausgestaltet sein.

Die Aufgabe der Destillationskolonne ist es, sicherzustellen, dass ein methanol- bzw. ethanolreiches Azeotrop abgeführt wird um die Verluste an zwangsläufig mit ausgetragenem Eduktester zu minimieren. Die Destillationskolonne kann ein, zwei oder mehr Trennstufen besitzen. Als Anzahl der Trennstufen wird die Anzahl der Böden bei einer Bodenkolonne oder die Anzahl der theoretischen Trennstufen im Fall einer Packungskolonne oder eine Kolonne mit Füllkörpern bezeichnet. Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz). Durch die umsatzabhängige Anpassung des Rücklaufverhältnisses gelingt es beispielsweise, bei Verwendung von Methylmethacrylat über weite Bereiche des Umsatzes einen Methanolanteil im Destillat einzustellen, der oberhalb von 60% liegt.

Zu den geeigneten Kondensatoren, die in der Anlage zur Durchführung der vorliegenden Umesterung enthalten sein kann, gehören unter anderem Platten- und Rohrbündelwärmetauscher.

Nach Beendigung der Umsetzung genügt das erhaltene (Meth)acrylat bereits vielfach den zuvor dargelegten hohen Anforderungen, so dass eine weitere Aufreinigung vielfach nicht notwendig ist. Zur weiteren Qualitätssteigerung und insbesondere der Katalysatorabtrennung kann die erhaltene Mischung durch bekannte Verfahren aufgereinigt werden.

Gemäß einer Ausgestaltung des erfindungsgemäßen Verfahrens kann die erhaltene Produktmischung durch Filtrationsverfahren aufgereinigt werden. Diese Verfahren sind aus dem Stand der Technik bekannt (W. Gösele, Chr. Alt in Ullmann's Encyclopedia of Industrial Chemistry, (6. Auflage), Verlag Wiley-VCH, Weinheim 2003 , Band 13, Seiten731und 746), wobei übliche Filtrationshilfsmittel, wie beispielsweise Bleicherde und/oder Aluminiumsilicat (Perlit) eingesetzt werden können. Beispielsweise können unter anderem kontinuierlich betreibbare Filter für eine Anschwemmfiltration oder Kerzenfilter verwendet werden.

Eine weitere Verbesserung der Qualität des Produkts kann beispielsweise durch eine Destillation des erhaltenen Filtrats erzielt werden. Wegen der Polymerisationsneigung des Monomeren sind Destillationsverfahren angeraten, bei denen die thermische Belastung des zu destillierenden Stoffes minimiert ist. Gut geeignet sind Vorrichtungen, bei denen das Monomer aus einer dünnen Schicht heraus kontinuierlich verdampft wird, wie Fallfilmverdampfer und Verdampfer mit einem rotierenden Wischersystem. Auch Kurzwegverdampfer können eingesetzt werden. Solche Vorrichtungen sind bekannt (Ullmanns Encyclopedia of Industrial Chemistry (6. Auflage), Verlag Wiley-VCH, Weinheim 2003, Band 36, Seite 505). So kann zum Beispiel ein kontinuierlicher Verdampfer mit rotierendem Wischersystem und aufgesetzter Kolonne eingesetzt werden. Die Destillation kann beispielsweise bei Druck im Bereich von 1 bis 40 mbar und einer Verdampfertemperatur von 120°C bis 150°C durchgeführt werden.

Nachfolgend soll die vorliegende Erfindung anhand einiger Beispiele erläutert werden.

### Beispiel 1

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 170 g (1,3 mol) 2-Ethylhexylalkohol, 455 g (4,55 mol) Methacrylsäuremethylester (MMA), 0,129 g Hydrochinonmonomethylether und 0,003 g 4-Hydroxy-2,2,6,6-tetramethylpiperidinyl-1-oxyl (Inhibitoren) und als Katalysator 62,5 g (10 Gew.-%) ®Amberlyst A26 (Rohm & Haas Comp./USA) in der Hydroxidform gemischt. Anschließend wurde der Ansatz bei einer Sumpftemperatur von 100 °C bis 123 °C 7 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert und bei 10 mbar vom MMA befreit.

Die erhaltene Ausbeute an 2-Ethylhexylmethacrylat betrug 117 g (45 % bezogen auf den eingesetzten 2-Ethylhexylalkohol). Die Reinheit des Produkts betrug 92,3% (gaschromatographisch bestimmt).

Ein Teil des Produkts wird vom Katalysator adsorptiv gebunden, der zweckmäßigerweise beim nächsten Ansatz wieder eingesetzt wird.

### Beispiel 2

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 606 g (6,06 mol) Methacrylsäuremethylester (MMA), 0,124 g Hydrochinonmonomethylether und 0,0025 g 4-Hydroxy-2,2,6,6-tetramethylpiperidinyl-1-oxyl (Inhibitoren) und als Katalysator 70,5 g (10 Gew.-%) ®Amberlyst A26 (Rohm & Haas Comp./USA) in der Hydroxidform gemischt. Der Ansatz wird unter Lufteinleitung zum Sieden erhitzt und azeotrop entwässert, bis eine Sumpftemperatur von 98 °C erreicht wird. Anschließend werden 99 g (1,1 mol) 1,4-Butandiol sowie eine dem abdestillierten MMA-Wassergemisch entsprechende Menge MMA (240g) zugegeben. Dann wurde der Ansatz bei einer Sumpftemperatur von 100 °C bis 113 °C 5 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert und bei 2 mbar vom MMA befreit.

Die erhaltene Ausbeute an 1,4-Butandioldimethacrylat betrug 133 g (53 % bezogen auf den eingesetzten Alkohol). Die Reinheit des Produkts betrug 87,5 % (gaschromatographisch bestimmt). Das Produkt enthält 10 % polymerisierbare Hochsieder, so dass der Gehalt an reaktiven Produktestern 97,5% beträgt.

Ein Teil des Produkts wird vom Katalysator adsorptiv gebunden, der zweckmäßigerweise beim nächsten Ansatz wieder eingesetzt wird.

### Beispiel 3

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 74,5g (1,2 mol) Ethylenglycol, 600 g (6 mol) Methacrylsäuremethylester (MMA), 0,119 g Hydrochinonmonomethylether und 0,0024 g 4-Hydroxy-2,2,6,6-tetramethylpiperidinyl-1-oxyl (Inhibitoren) und als Katalysator 67,5 g (10 Gew.-%) ®Amberlyst A26 (Rohm & Haas Comp./USA) in der Hydroxidform gemischt. Anschließend wurde der Ansatz bei einer Sumpftemperatur von 100 °C bis 121 °C 5 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert, der Katalysator mit 100g MMA extrahiert und Produkt und Extrakt zusammen bei 10 mbar vom MMA befreit.

Die erhaltene Ausbeute an Ethylenglycoldimethacrylat betrug 178 g (74,8 % bezogen auf das eingesetzte Ethylenglykol). Die Reinheit des Produkts betrug 89,8% (gaschromatographisch bestimmt). Das Produkt enthält 5,7 % polymerisierbare Hochsieder sowie 0,47% Hydroxyethylmethacrylat, so dass der Gehalt an reaktiven Produktestern 96% beträgt. Die Pt-Co-Farbzahl beträgt 44.

Ein Teil des Produkts wird vom Katalysator adsorptiv gebunden, der zweckmäßigerweise beim nächsten Ansatz wieder eingesetzt wird.

### Beispiel 4

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 495 g (4,95 mol) Methacrylsäuremethylester (MMA), 0,06 g Phenothiazin und 0,0045 g 4-Hydroxy-2,2,6,6-tetramethylpiperidinyl-1-oxyl (Inhibitoren) und als Katalysator 78,4 g (10 Gew.-%) ®Amberlyst A26 (Rohm & Haas Comp./USA) in der Hydroxidform gemischt. Der Ansatz wird unter Lufteinleitung zum Sieden erhitzt und azeotrop entwässert, bis eine Sumpftemperatur von 98 °C erreicht wird. Anschließend werden 285g (0,9 mol) Dianol® 220 (zweifach ethoxyliertes Bisphenol A der Fa. Seppic, Frankreich) sowie eine dem abdestillierten MMA-Wassergemisch entsprechende Menge MMA (310g) zugegeben. Dann wurde der Ansatz bei einer Sumpftemperatur von 100 °C bis 120 °C 8,5 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert, der Katalysator mit 100g MMA extrahiert und Produkt und Extrakt zusammen bei 3 mbar vom MMA befreit.

Die erhaltene Ausbeute an ethoxyliertem Bisphenol A-dimethacrylat betrug 338 g (83 % bezogen auf den eingesetzten Rohstoff). Die Reinheit des Produkts betrug 83,4% (gaschromatographisch bestimmt). Das Produkt enthält 13,9 % polymerisierbare Hochsieder, so dass der Gehalt an reaktiven Produktestern 97,3% beträgt. Die Pt-Co-Farbzahl beträgt 30.

Ein Teil des Produkts wird vom Katalysator adsorptiv gebunden, der zweckmäßigerweise beim nächsten Ansatz wieder eingesetzt wird.

### Beispiel 5

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 285g (0,9 mol) Newpol® BPE 20-F (zweifach ethoxyliertes Bisphenol A der Fa. Sanyo, Japan), 495 g (4,95 mol) Methacrylsäuremethylester (MMA), 0,06 g Phenothiazin und 0,0045 g 4-Hydroxy-2,2,6,6-tetramethylpiperidinyl-1-oxyl (Inhibitoren) und als Katalysator 78,4 g (10 Gew.-%) gebrauchter Katalysator aus Beispiel 4 gemischt. Dann wurde der Ansatz bei einer Sumpftemperatur von 100 °C bis 120 °C 8 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert, der Katalysator mit 100g MMA extrahiert und Produkt und Extrakt zusammen bei 3 mbar vom MMA befreit.

Die erhaltene Ausbeute an ethoxyliertem Bisphenol A-dimethacrylat betrug 350 g (86 % bezogen auf den eingesetzten Rohstoff). Die Reinheit des Produkts betrug 70,8% (gaschromatographisch bestimmt). Das Produkt enthält 24,5 % polymerisierbare Hochsieder, so dass der Gehalt an reaktiven Produktestern 95,3% beträgt. Die Pt-Co-Farbzahl beträgt 25.

Ein Teil des Produkts wird vom Katalysator adsorptiv gebunden, der zweckmäßigerweise beim nächsten Ansatz wieder eingesetzt wird.

### Beispiel 6

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 170 g (1,3 mol) 2-Ethylhexylalkohol, 455 g (4,55 mol) Methacrylsäuremethylester (MMA), 0,129 g Hydrochinonmonomethylether und 0,003 g 4-Hydroxy-2,2,6,6-tetramethylpiperidinyl-1-oxyl (Inhibitoren) und als Katalysator 80 g des gebrauchten Katalysators aus Beispiel 1 gemischt. Anschließend wurde der Ansatz bei einer Sumpftemperatur von 100 °C bis 123 °C 8 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert und bei 10 mbar vom MMA befreit.

Die erhaltene Ausbeute an 2-Ethylhexylmethacrylat betrug 156 g (60 % bezogen auf den eingesetzten 2-Ethylhexylalkohol). Die Reinheit des Produkts betrug 91,8% (gaschromatographisch bestimmt).

Ein Teil des Produkts wird vom Katalysator adsorptiv gebunden, der zweckmäßigerweise beim nächsten Ansatz wieder eingesetzt wird.

### Beispiel 7

Beispiel 7 wurde im Wesentlichen entsprechend dem Beispiel 1 durchgeführt, wobei jedoch 62,5 g Ambersep® 900 (Rohm & Haas Comp./USA) in der Hydroxidform eingesetzt wurden.

Die erhaltene Ausbeute an 2-Ethylhexylmethacrylat betrug 120 g (46 % bezogen auf den eingesetzten 2-Ethylhexylalkohol). Die Reinheit des Produkts betrug 93% (gaschromatographisch bestimmt).

Ein Teil des Produkts wird vom Katalysator adsorptiv gebunden, der zweckmäßigerweise beim nächsten Ansatz wieder eingesetzt wird.

### Beispiel 8

100 g ®Amberlyst A26 (Rohm & Haas Comp./USA) in der Chloridform werden mit Hilfe von vollentsalztem Wasser in eine Chromatographiesäule gefüllt und mit 10% iger Natriumcarbonatlösung solange versetzt, bis das ablaufende Eluat chloridfrei ist. Dann wird mit vollentsalztem Wasser nachgewaschen, bis das ablaufende Eluat neutral reagiert. Man wäscht mit Methanol nach und trocknet den erhaltenen ®Amberlyst A26 in der Carbonatform an der Luft.

In einem 1000 ml Vierhalsrundkolben, der mit einem Säbelrührer mit Rührhülse und Rührmotor, Lufteinleitung, Sumpfthermometer und einer Destillationskolonne mit Kolonnenkopf und Rücklaufteiler ausgestattet war, wurden 170 g (1,3 mol) 2-Ethylhexylalkohol, 455 g (4,55 mol) Methacrylsäuremethylester (MMA), 0,129 g Hydrochinonmonomethylether und 0,003 g 4-Hydroxy-2,2,6,6-tetramethylpiperidinyl-1-oxyl (Inhibitoren) und als Katalysator 62,5 g (10 Gew.-%) ®Amberlyst A26 (Rohm & Haas Comp./USA) in der Carbonatform gemischt. Anschließend wurde der Ansatz bei einer Sumpftemperatur von 100 °C bis 123 °C 8,5 Stunden unter Lufteinleitung gerührt, wobei Methanol und Methylmethacrylat abdestilliert wurden. Nach Beendigung der Reaktion wurde das Produkt zur Katalysatorabtrennung durch ein Faltenfilter filtriert und bei 9 mbar vom MMA befreit.

Die erhaltene Ausbeute an 2-Ethylhexylmethacrylat betrug 125 g (48 % bezogen auf den eingesetzten 2-Ethylhexylalkohol). Die Reinheit des Produkts betrug 94% (gaschromatographisch bestimmt).

Ein Teil des Produkts wird vom Katalysator adsorptiv gebunden, der zweckmäßigerweise beim nächsten Ansatz wieder eingesetzt wird.

## Patentansprüche

1. Verfahren zur Herstellung von (Meth)acrylsäureestern, umfassend die Umesterung eines als Ausgangsverbindung eingesetzten Esters mit einem niedrigeren Siedepunkt als der durch Umesterung erhaltenen Ester der (Meth)acrylsäure mit einem Eduktalkohol in Gegenwart eines Katalysators, **dadurch gekennzeichnet, dass** die Umesterung durch einen basischen Ionenaustauscher katalysiert wird.

2. Verfahren gemäß Anspruch 1, **dadurch gekennzeichnet dass** ein stark basischer Ionenaustauscher mit quartären Stickstaff-Gruppen eingesetzt wird.

3. Verfahren gemäß Anspruch 1 oder 2, **dadurch gekennzeichnet, dass** Methyl(meth)acrylat und/oder Ethyl(meth)acrylat als niedrig siedender Ester der (Meth)acrylsäure eingesetzt wird.

4. Verfahren gemäß mindestens einem der vorhergehenden Ansprüche, **dadurch gekennzeichnet, dass** der aus dem niedrig siedenden Ester der (Meth)acrylsäure freigesetzte Alkohol durch Destillation abgetrennt wird.

5. Verfahren gemäß mindestens einem der vorhergehenden Ansprüche, **dadurch gekennzeichnet, dass** ein einwertiger Alkohol als Eduktalkohol eingesetzt wird.

6. Verfahren gemäß Anspruch 5, **dadurch gekennzeichnet, dass** als Eduktalkohol Butanol, Hexanol, 3-Methylbutanol, Heptanol, 2-Ethylhexylalkohol, 2-tert.-Butylheptanol, Octanol, 3-iso-Propylheptanol, Nonanol, Decanol, Undecanol, 5-Methylundeanol, Dodecanol, 2-Methyldodecanol, Tridecanol, 5-Methyltridecanol, Tetradecanol, Pentadecanol, Hexadecanol, 2-Methylhexadecanol, Heptadecanol, 5-iso-Propylheptadecanol, 4-tert.-Butyloctadecanol, 5-Ethyloctadecanol, 3-iso-Propyloctadecanol, Octadecanol, Nonadecanol, Eicosanol, Cetyleicosanol, Stearyleicosanol, Docosanol und/oder Eicosyltetratriacontanol eingesetzt wird.

7. Verfahren gemäß Anspruch 1, **dadurch gekennzeichnet, dass** ein mehrwertiger Alkohol als Eduktalkohol eingesetzt wird.

8. Verfahren gemäß Anspruch 7, **dadurch gekennzeichnet, dass** als Eduktalkohol Ethylenglycol, Trimethylolpropan, 1,2-Propandiol, 1,3-Propandiol, 1,3-Butandiol, 1,4-Butandiol, ethoxyliertes Bisphenol A, 1,6-Hexandiol und/oder Pentaerythrit eingesetzt wird.

9. Verfahren gemäß mindestens einem der vorhergehenden Ansprüche, **dadurch gekennzeichnet, dass** die Reaktionsmischung höchstens 0,5 Gew.-% Wasser umfasst.

10. Verfahren gemäß Anspruch 9, **dadurch gekennzeichnet, dass** die Reaktionsmischung höchstens 0,05 Gew.-% Wasser umfasst.

11. Verfahren gemäß mindestens einem der vorhergehenden Ansprüche, **dadurch gekennzeichnet, dass** das in der Reaktionsmischung vorhandene Molverhältnis von niedrig siedendem Ester der (Meth)acrylsäure zu Eduktalkohol während der Umsetzung durch Zugabe von niedrig siedenden Ester der (Meth)acrylsäure um mindestens 40% erhöht wird.

12. Verfahren gemäß mindestens einem der vorhergehenden Ansprüche, **dadurch gekennzeichnet, dass** eine Mischung abgetrennt wird, die Methylmethacrylat und Methanol enthält.

13. Verfahren gemäß mindestens einem der vorhergehenden Ansprüche, **dadurch gekennzeichnet, dass** während der Umesterung Methylmethacrylat zugegeben wird.

14. Verfahren gemäß mindestens einem der vorhergehenden Ansprüche, **dadurch gekennzeichnet, dass** die Reaktionszeit im Bereich von 3 bis 20 Stunden liegt.

15. Verfahren gemäß mindestens einem der vorhergehenden Ansprüche, **dadurch gekennzeichnet, dass** der basische Ionenaustauscher Hydroxidionen und/oder Carbonationen aufweist.

16. Verfahren gemäß mindestens einem der vorhergehenden Ansprüche, **dadurch gekennzeichnet, dass** das Gewichtsverhältnis von Eduktalkohol zu niedrig siedenden Ester der (Meth)acrylsäure im Bereich von 2:1 bis 1:10 liegt.

17. Verfahren gemäß mindestens einem der vorhergehenden Ansprüche, **dadurch gekennzeichnet, dass** die Umsetzung bei einem Druck im Bereich von 200 bis 2000 mbar erfolgt.

18. Verfahren gemäß mindestens einem der vorhergehenden Ansprüche, **dadurch gekennzeichnet, dass** die Umsetzung bei einer Temperatur im Bereich von 50°C bis 140°C erfolgt.

19. Verfahren gemäß mindestens einem der vorhergehenden Ansprüche, **dadurch gekennzeichnet, dass** die Umesterung in Gegenwart eines Polymerisationsinhibitors erfolgt.

20. Verfahren gemäß mindestens einem der vorhergehenden Ansprüche, **dadurch gekennzeichnet, dass** das Verfahren kontinuierlich durchgeführt wird.

21. Verfahren gemäß mindestens einem der vorhergehenden Ansprüche, **dadurch gekennzeichnet, dass** ein bereits in der Umesterungsreaktion eingesetzter basischer Ionentauscher als Katalysator eingesetzt wird.

22. Verfahren gemäß mindestens einem der vorhergehenden Ansprüche, **dadurch gekennzeichnet, dass** der gebrauchte basische Ionentauscher durch Behandeln mit einer wässrigen Alkalimetallhydroxid- oder -carbonatlösung regeneriert wird.

## Claims

1. Process for preparing (meth)acrylic esters, comprising the transesterification of an ester which has a lower boiling point than the ester of (meth)acrylic acid obtained by transesterification and is used as starting compound with a reactant alcohol in the presence of a catalyst, **characterized in that** the transesterification is catalysed by a basic ion exchanger.

2. Process according to Claim 1, **characterized in that** a strongly basic ion exchanger having quaternary nitrogen groups is used.

3. Process according to Claim 1 or 2, **characterized in that** methyl (meth)acrylate and/or ethyl (meth)acrylate is/are used as low-boiling ester(s) of (meth)acrylic acid.

4. Process according to at least one of the preceding claims, **characterized in that** the alcohol released from the low-boiling ester of (meth)acrylic acid is removed by distillation.

5. Process according to at least one of the preceding claims, **characterized in that** a monohydric alcohol is used as the reactant alcohol.

6. Process according to Claim 5, **characterized in that** the reactant alcohol used is butanol, hexanol, 3-methylbutanol, heptanol, 2-ethylhexyl alcohol,
2-tert-butylheptanol, octanol, 3-isopropylheptanol, nonanol, decanol, undecanol, 5-methylundecanol, dodecanol, 2-methyldodecanol, tridecanol, 5-methyltridecanol, tetradecanol, pentadecanol, hexadecanol, 2-methylhexadecanol, heptadecanol, 5-isopropylheptadecanol, 4-tert-butyloctadecanol, 5-ethyloctadecanol, 3-isopropyloctadecanol, octadecanol, nonadecanol, eicosanol, cetyleicosanol, stearyleicosanol, docosanol and/or eicosyltetratriacontanol.

7. Process according to Claim 1, **characterized in that** a polyhydric alcohol is used as the reactant alcohol.

8. Process according to Claim 7, **characterized in that** the reactant alcohol used is ethylene glycol, trimethylolpropane, 1,2-propanediol, 1,3-propanediol, 1,3-butanediol, 1,4-butanediol, ethoxylated bisphenol A, 1,6-hexanediol and/or pentaerythritol.

9. Process according to at least one of the preceding claims, **characterized in that** the reaction mixture comprises at most 0.5% by weight of water.

10. Process according to Claim 9, **characterized in that** the reaction mixture comprises at most 0.05% by weight of water.

11. Process according to at least one of the preceding claims, **characterized in that** the molar ratio of low-boiling ester of (meth)acrylic acid to reactant alcohol present in the reaction mixture is increased during the reaction by at least 40% by adding low-boiling ester of (meth)acrylic acid.

12. Process according to at least one of the preceding claims, **characterized in that** a mixture which comprises methyl methacrylate and methanol is removed.

13. Process according to at least one of the preceding claims, **characterized in that** methyl methacrylate is added during the transesterification.

14. Process according to at least one of the preceding claims, **characterized in that** the reaction time is in the range from 3 to 20 hours.

15. Process according to at least one of the preceding claims, **characterized in that** the basic ion exchanger comprises hydroxide ions and/or carbonate ions.

16. Process according to at least one of the preceding claims, **characterized in that** the weight ratio of reactant alcohol to low-boiling ester of (meth)acrylic acid is in the range from 2:1 to 1:10.

17. Process according to at least one of the preceding claims, **characterized in that** the reaction is effected at a pressure in the range from 200 to 2000 mbar.

18. Process according to at least one of the preceding claims, **characterized in that** the reaction is effected at a temperature in the range from 50°C to 140°C.

19. Process according to at least one of the preceding claims, **characterized in that** the transesterification is effected in the presence of a polymerization inhibitor.

20. Process according to at least one of the preceding claims, **characterized in that** it is performed continuously.

21. Process according to at least one of the preceding claims, **characterized in that** a basic ion exchanger already used in the transesterification reaction is used as the catalyst.

22. Process according to at least one of the preceding claims, **characterized in that** the spent basic ion exchanger is regenerated by treating with an aqueous alkali metal hydroxide or carbonate solution.

## Revendications

1. Procédé pour la production d'esters d'acide (méth)acrylique, comprenant la transestérification d'un ester, utilisé comme composé de départ, ayant un plus bas point d'ébullition que l'ester de l'acide (méth)acrylique avec un alcool de départ, obtenu par transestérification, en présence d'un catalyseur, **caractérisé en ce que** la transestérification est catalysée par un échangeur d'ions basique.

2. Procédé selon la revendication 1, **caractérisé en ce qu'**on utilise un échangeur d'ions fortement basique comportant des groupes à azote quaternaire.

3. Procédé selon la revendication 1 ou 2, **caractérisé en ce qu'**on utilise comme ester à bas point d'ébullition de l'acide (méth)acrylique le (méth)acrylate de méthyle et/ou le (méth)acrylate d'éthyle.

4. Procédé selon au moins l'une quelconque des revendications précédentes, **caractérisé en ce qu'**on sépare par distillation l'alcool libéré de l'ester à bas point d'ébullition de l'acide (méth)acrylique.

5. Procédé selon au moins l'une quelconque des revendications précédentes, **caractérisé en ce qu'**on utilise comme alcool de départ un alcool monohydrique.

6. Procédé selon la revendication 5, **caractérisé en ce qu'**on utilise comme alcool de départ le butanol, l'hexanol, le 3-méthylbutanol, l'heptanol, l'alcool 2-éthylhexylique, le 2-tert-butylheptanol, l'octanol, le 3-isopropylheptanol, le nonanol, le décanol, l'undécanol, le 5-méthylundécanol, le dodécanol, le 2-méthyldodécanol, le tridécanol, le 5-méthyltridécanol, le tétradécanol, le pentadécanol, l'hexadécanol, le 2-méthylhexadécanol, l'heptadécanol, le 5-isopropylheptadécanol, le 4-tert-butyloctadécanol, 1 e 5-éthyloctadécanol, le 3-isopropyloctadécanol, l'octadécanol, le nonadécanol l'eicosanol, le cétyleicosanol, le stéaryleicosanol, le docosanol et/ou l'eicosyltétratriacontanol.

7. Procédé selon la revendication 1, **caractérisé en ce que** qu'on utilise comme alcool de départ un alcool polyhydrique.

8. Procédé selon la revendication 7, **caractérisé en ce qu'**on utilise comme alcool de départ l'éthylèneglycol, le triméthylolpropane, le 1,2-propanediol, le 1,3-propanediol, le 1,3-butanediol, le 1, 4-butanediol, le bisphénol A éthoxylé, le 1,6-hexanediol et/ou le pentaérythritol.

9. Procédé selon au moins l'une quelconque des revendications précédentes, **caractérisé en ce que** le mélange réactionnel comprend au maximum 0,5 % en poids d'eau,

10. Procédé selon la revendication 9, **caractérisé en ce que** le mélange réactionnel comprend au maximum 0,05 % en poids d'eau.

11. Procédé selon au moins l'une quelconque des revendications précédentes, **caractérisé en ce que** le rapport molaire d'ester à bas point d'ébullition de l'acide (méth)acrylique à alcool de départ, présent dans le mélange réactionnel, est augmenté d'au moins 40 % pendant la réaction, par addition d'ester à bas point d'ébullition de l'acide (méth)acrylique.

12. Procédé selon au moins l'une quelconque des revendications précédentes, **caractérisé en ce qu'**on sépare un mélange qui contient du méthacrylate de méthyle et du méthanol.

13. Procédé selon au moins l'une quelconque des revendications précédentes, **caractérisé en ce que** pendant la transestérification on ajoute du méthacrylate de méthyle.

14. Procédé selon au moins l'une quelconque des revendications précédentes, **caractérisé en ce que** le temps de réaction se situe dans la plage de 3 à 20 heures.

15. Procédé selon au moins l'une quelconque des revendications précédentes, **caractérisé en ce que** l'échangeur d'ions basique comporte des ions hydroxyde et/ou des ions carbonate.

16. Procédé selon au moins l'une quelconque des revendications précédentes, **caractérisé en ce que** le rapport pondéral d'alcool de départ à ester à bas point d'ébullition de l'acide (méth)acrylique se situe dans la plage de 2:1 à 1:10.

17. Procédé selon au moins l'une quelconque des revendications précédentes, **caractérisé en ce que** la réaction s'effectue sous une pression dans la plage de 200 à 2 000 mbars.

18. Procédé selon au moins l'une quelconque des revendications précédentes, **caractérisé en ce que** la réaction s'effectue à une température dans la plage de 50 °C à 140 °C.

19. Procédé selon au moins l'une quelconque des revendications précédentes, **caractérisé en ce que** la transestérification s'effectue en présence d'un inhibiteur de polymérisation.

20. Procédé selon au moins l'une quelconque des revendications précédentes, **caractérisé en ce que** le procédé est effectué en continu.

21. Procédé selon au moins l'une quelconque des revendications précédentes, **caractérisé en ce qu'**on utilise comme catalyseur un échangeur d'ions basique déjà utilisé dans la réaction de transestérification.

22. Procédé selon au moins l'une quelconque des revendications précédentes, **caractérisé en ce qu'**on régénère l'échangeur d'ions basique utilisé, par traitement par une solution aqueuse d'hydroxyde ou de carbonate de métal alcalin.
